# EUROPEAN PATENT SPECIFICATION

(11) **EP 2 687 510 B1**
(45) Date of publication and mention of the grant of the patent: **30.03.2016**
(21) Application number: 11861242.3
(22) Date of filing: 07.06.2011
(51) Int. Cl.: C07D 213/61

(54) **METHOD FOR PREPARING 2,3-DICHLOROPYRIDINE**
VERFAHREN ZUR HERSTELLUNG VON 2,3-DICHLORPYRIDIN
PROCÉDÉ DE PRÉPARATION DE 2,3-DICHLOROPYRIDINE

(30) Priority: 13.03.2011 CN 201110060563
(43) Date of publication of application: 22.01.2014
(73) Proprietor: Lianhe Chemical Technology Co., Ltd., Zhejiang 318020 (CN); Jiangsu Lianhe Chemical Technology Co., Ltd, Yancheng, Jiangsu 224600 (CN)
(72) Inventor: WANG, Ping, Taizhou Zhejiang 318020 (CN); FAN, Xiaobin, Taizhou Zhejiang 318020 (CN); ZHANG, Juntao, Taizhou Zhejiang 318020 (CN); YE, Fangsheng, Taizhou Zhejiang 318020 (CN)
(74) Representative: Hannke, Christian
(86) International application number: PCT/CN2011/075369
(87) International publication number: WO 2012/122746

(56) References cited:
- EP-A1- 0 300 430
- CN-A- 101 774 966
- JP-A- 1 193 246
- CHEN XU ET AL.: 'The Synthesis of 2,3,6-Trichloropyridine' SPECIALITY PETROCHEMICALS 2000, pages 38 - 40, XP008169967
- FENG CUN ET AL.: 'One-pot Synthesis of 2,3-Dichloropyridine' FINE CHEMICAL INTERMEDIATES vol. 38, no. 5, 2008, pages 19 - 21, 45, XP009178514

## Description

### Technical Field

The present invention relates to a chemical synthesis method, specifically relates to a method for preparing 2, 3-dichloropyridine which is an important fine-chemical intermediate and widely used in the field of pharmaceuticals and pesticides.

### Prior Art

2,3-dichloropyridine is a kind of important fine-chemical intermediate, which is widely used in the field of pharmaceuticals and pesticides. Having been reported both at home and abroad, methods for preparing 2,3-dichloropyridine, having different chemical processes, are mainly as follows:
1. Both patent US 4515953 and patent SU 652177 disclosed a process for chlorinating pyridine or pyridine hydrochloride in liquid phase. However, the method gave the mixture of many other chlorinated pyridine except for 2, 3-dichloropyridine, and it also needed multistep purification and extraction in order to obtain 2,3-dichloropyridine.
2. Patent US 62242631 and a research paper in J. Org. Chem. 1988, 53, 12, 2858-9 reported by Bay et. al. disclosed a method for preparing 2,3-dichloropyridine which is halogenating 2-chloro-3-nitro-pyridine with phenylphosphorous tetrachloride (PPTC) and benzenephosphorours dichloride (BPOD).
3. The preparation method of several chloropyridine derivatives, that treating 2-chloro-3-aminopyridine with sodium nitrite, copper and hydrochloride acid, has been reported in the Rec. Des Trav. Chimi. Des Pays-Bas et de la Belgique, 1950, 69, 673-99 by Den Hertog et. al. However, it didn't relate to the extraction and purification steps of 2,3-dichloropyridine.
4. Patent WO 2005070888 disclosed a four-step method for preparing 2,3-dichloropyridine, wherein, in the aqueous solution of hydrochloric acid, 3-amino-2-chloropyridine contacted with alkali metal nitrite to generate diazonium salts; and then 2,3-dichloropyridine was given after decomposing in the presence of copper catalyst (wherein at least about 50% of the copper is the oxidation state of copper (II))and optional organic solvent. In the method the 3-amino-2-chloropyridine was prepared by three steps, including Hofmann rearrangement of niacinamide to give 3-aminopyridine, 3-aminopyridine in contact with hydrochloric acid to give 3-aminopyridine hydrochloride; the chlorination of 3-aminopyridine hydrochloride with a chlorinating agent, i.e. chlorine, or a mixture of hydrochloric acid and hydrogen peroxide, to generate 3-amino-2-chloropyridine.
5. Patent CN 101302190 and Patent CN 1807414 disclosed that 3-aminopyridine used as raw material was diazotized with sodium nitrite in the presence of concentrated hydrochloric acid and hydrogen peroxide to give a diazonium salt which was then chlorinated to give 2,3-dichloropyridine. All the processes of the invention is completed in only one reaction pot without purifying and separating intermediates, and conventional isolation and purification methods were used.

In summary, in the prior art, the preparing method for 2,3-dichloropyridine was mainly used 3-aminopyridine as starting raw material, via chlorination, diazotization and hydrolyzing chloride. The method related many steps and has a high production cost for using 3-aminopyridine as starting raw materials, and furthermore, large amounts of waste would be let out during the processes of diazotization and chlorination, which would seriously pollute the environment, and limit the scale of industrial production of 2,3-dichloropyridine.

### Contents of the Invention

Accordingly, to solve the above-mentioned problems existing in the method for preparing of 2,3-dichloropyridine in the prior art which are high raw material cost, too many synthesizing steps and complicated types of the reaction etc., provides the present invention a method for preparing 2,3-dichloropyridine which has cheap raw materials, simple processes, high yields and is environmental friendly and suitable for manufacturing in an industrial scale.

The present invention provides a method for preparing 2,3-dichloropyridine which comprises the following steps:
A. chlorination: mixing the staring raw material 2,6-dichloropyridine with a catalyst, then warming up and introducing chlorine gas to chlorinate, when the reaction has finished, cooling and fractionating under reduced pressure to give 2,3,6-trichloropyridine;
   Further,
   The catalyst of said chlorination is selected from a kind of common Lewis acid: sulfur trioxide, boron trifluoride, ferric chloride or aluminum chloride, the amount of the catalyst is 0.01 to 1.00 times the molar amount of 2,6-dichloropyridine, preferably 0.01 to 0.50 times.

The temperature of said chlorination is generally 50∼300°C, preferably 100∼200°C.

The distillate with low concentration gained during the process of fractionating of said chlorination can be recycled for the next batch of reaction and rectification.
B. hydrogenation: adding 2,3,6-trichloropyridine gained by the chlorination, an acid-binding agent, a metal catalyst and organic solvent into a reactor, under the pressure range of 0-10MPa, introducing hydrogen gas to hydrogenate after warming, when the pH value of the reaction solution has reached 4∼8, stopping introducing the hydrogen gas. The molar ratio of said 2,3,6-trichloropyridine to the acid-binding agent is 1:0.1∼0.5, preferably 1:0.3∼0.5. The scope of pressure of the hydrogenation is preferably 0∼5Mpa. The temperature of the hydrogenation is generally 20-200°C, preferably 20-150°C.

Further,
The acid-binding agent of the hydrogenation is selected from organic base or inorganic base compound. Said organic base compound is selected from pyridine, triethylamine, alkaline metal salts of alcohols, alkyl lithium compound or N-lithium compound; preferably pyridine or triethylamine.

Wherein, the alkaline metal salts of alcohols is selected from sodium methylate, potassium ethylate, or potassium tert-butoxide; the alkyl lithium compound is selected from butyl lithium or phenyl lithium; the N-lithium compound is selected from lithium diisopropyl amide or lithium hexamethyldisilazide; said alkaline metal hydroxides is selected from sodium hydroxide or potassium hydroxide; the alkaline metal carbonates and bicarbonates is selected from sodium carbonate, potassium carbonate, sodium bicarbonate or potassium bicarbonate; the acetates is selected from sodium acetate or potassium acetate. Said inorganic base compound is selected from the group consisting of alkaline metal hydroxides, alkaline metal carbonates and bicarbonates, acetates.

The metal catalyst of said hydrogenation is selected from mono-metallic catalyst, multi-metal catalyst, supported metal catalyst or non-supported metal catalyst. The mono-metallic catalyst is selected from platinum, palladium, cobalt or nickel; the multi-metal catalyst is selected from one of the binary alloy catalysts which is copper-nickel, copper- palladium, palladium-silver, platinum-gold or platinum-copper; the supported metal catalyst is selected from platinum-carbon or palladium-carbon; the non-supported metal catalyst is selected from Raney nickel, Raney copper or Raney cobalt; preferably platinum-carbon, palladium-carbon, Raney nickel or Raney copper.

The organic solvent of the hydrogenation is selected from esters, ethers, aromatic hydrocarbons, nitriles, or any mixture of them; the esters is selected from methyl acetate, ethyl acetate or butyl acetate; the ethers is selected from diethyl ether, methyl tert-butyl ether, tetrahydrofuran or dioxane, the aromatic hydrocarbons is selected from benzene, toluene or chlorobenzene, the nitriles is selected from acetonitrile or propionitrile; preferably toluene or ethyl acetate.
C. post-processing: cooling the aforementioned reaction solution to room temperature, adding water to dissolve the hydrochloride of acid-binding agent, filtering, and layering the filtrate, separating the water layer, extracting the organic layer with aqueous acid at least three times, then combining the aqueous acid layer after the extraction and adding water to dilute, crystallizing, filtering to give wet 2,3-dichloropyridine, drying the wet product to give the product of 2,3-dichloropyridine.

Further,
In the post-processing, the catalyst gained by filtration after water is added to dissolve the hydrochloride of acid-binding agent can be recycled.

In the post-processing, when replenishing 2,3,6-trichloropyridine and organic solvent, the organic solvent layer can be recycled as material for the next batch.

In the post-processing, the aqueous acid is selected from water-soluble inorganic acid or organic acid. The inorganic acid is selected from hydrochloric acid, sulfuric acid, carbonic acid, phosphoric acid or nitric acid. The organic acid is selected from low-level carboxylic acid: formic acid, acetic acid, propionic acid, n-butyric acid or n-pentanoic acid.

In the post-processing, the molar ratio of the aqueous acid to 2,3,6-trichloropyridine is 1∼5:1.

Further, in the post-processing, the organic acid can also be selected from salicylic acid or benzenesulfonic acid.

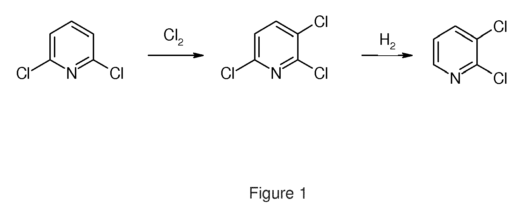

The equation of the present invention (refer to figure 1): 2,3,6-tridichloropyridine is given by the chlorination with the starting raw material 2,6-dichloropyridine and chlorine gas, then hydrogenating 2,3,6-trichloropyridine to give 2, 3-dichloropyridine. Thus, it can be seen that the present invention adopts efficient recycling techniques, has a low cost and a novel and simple route and is suitable for industrial manufacturing.

### Description of Drawings

Figure 1 is the equation of the present invention.

The invention was further described by embodiments which illustrate details of the invention, but the scope of the invention is not intended to be limited.

### Embodiments

### Embodiment 1

### Chlorination

1480.0g 2,6-dichloropyridine and 89.2g anhydrous FeC13 are weighed, mixed and added into a 2000mL four-necked flask. When the mixture was heated to 100∼120°C, chlorine gas is introduced until the reaction is carried out adequately, then cooled to 100°C, fractionated under reduced pressure. The product fraction is collected at the top temperature of 118∼124°C under the pressure of -0.1MPa. The distillate with low concentration gained during the process of fractionating can be recycled for the next batch of reaction and fractionating. Finally, 1715.0g 2,3,6-trichloropyridine is gained when the distillate is recycled, and the total yield is 94.0%, the purity is greater than or equal to 99.5%.

### Hydrogenation

557.8g 2,3,6-trichloropyridine, which is obtained by the chlorination, 123.5g triethylamine, 8.4g palladium-carbon and 1673g toluene are added to a reactor for one time, then heated to 60∼80°C, and hydrogen gas is introduced to proceed hydrogenating. When pH value of the reaction solution reaches 4∼8, stopping introducing the hydrogen gas, and the hydrogenation finishes.

### Post-processing

After the aforementioned reaction solution was cooled to room temperature, 420g water is added for dissolving triethylamine hydrochloride. Then the reaction solution is filtered, and the cake which is palladium carbon washed by 30g water and 30g toluene can be recycled. The filtrate is allowed to stand stratification, and then the toluene layer is extracted by 200g hydrochloric acid at least three times after the water was separated. The extracted solution can be recycled for the next batch with replenishing 2,3,6-trichloropyridine and toluene. The hydrochloride layers are combined and diluted by 1630g water after extraction, and then solid is crystallized. The solid is washed by 100g water (the waste water can be recycled for extraction) after filtration, then wet 2,3-dichloropyridine is given. Dry product of 2,3-dichloropyridine is given by drying the wet product. Finally, 387.7g product is totally gained when the waste water is recycled, and the yield is 85.7%, the purity is greater than or equal to 99.5%.

### Embodiment 2

### Chlorination

299.2g 2,6-dichloropyridine, 533.7g the recycled distillate (the distillate contains 86.1% 2,6-dichloropyridine and 9% 2,3,6-trichloropyridine), and 36.5g anhydrous AlCl3 are weighed, mixed and added into a 1000mL four-necked flask. When the mixture is heated to 120∼140°C, chlorine gas is introduced until the reaction proceeds adequately, then cooled to 100°C, fractionated under reduced pressure. The product fraction is collected at the top temperature of 118∼124°C under the pressure of -0.1MPa. The distillate with low concentration gained during the process of fractionating can be recycled for the next batch of reaction and fractionating. Finally, 890.7g 2,3,6-trichloropyridine is gained when the distillate is recycled, and the total yield is 95.2%, the purity is greater than or equal to 99.5%.

### Hydrogenation

1340g solution of 2,3,6-trichloropyridine in toluene gained in embodiment 1, 410.5g 2,3,6-trichloropyridine and 348g toluene (convert into: 562.7g 2,3,6-trichloropyridine with an addition of 85.1g pyridine and 5.6g palladium-carbon) can be added into a reactor for one time. When the mixture is heated to 60∼80°C, hydrogen gas is introduced until pH value reaches 4∼8 and the hydrogenating finishes.

### Post-processing

After the aforementioned reaction solution was cooled to room temperature, 400g water is added for dissolving pyridine hydrochloride. Then the reaction solution is filtered, and the cake which is palladium carbon washed by 30g water and 30g toluene can be recycled. The filtrate is allowed to stand stratification, and then the toluene layer is extracted by 200g hydrochloride three times after the water layer was separated. The extracted solution can be recycled for the next batch with replenishing 2,3,6-trichloropyridine and toluene. The hydrochloric acid layers are combined and diluted by 1650g water after extraction, and the solid is participated, filtered, then washed by 120g water (the waste water can be recycled for extraction) to give wet 2,3-dichloropyridine. Dry product of 2,3-dichloropyridine is given by drying the wet product. Finally, 388.9g product is totally gained when the waste water is recycled, and the yield is 85.4%, the purity is greater than or equal to 99.5%.

## Claims

1. A method for preparing 2,3-dichloropyridine, which comprises the following steps:
(1) chlorination: mixing the starting raw material 2,6-dichloropyridine with a catalyst, then warming up and introducing chlorine gas to chlorinate , when the reaction has finished, cooling and fractionating under reduced pressure to give 2,3,6-trichloropyridine;
(2) hydrogenation: adding 2,3,6-trichloropyridine gained by the chlorination, an acid-binding agent, a metal catalyst and organic solvent into a reactor, under the pressure range of 0∼10MPA, introducing hydrogen gas to hydrogenate after warming, when the pH value of the reaction solution has reached 4∼8, stopping introducing the hydrogen gas; the molar ratio of said 2,3,6-trichloropyridine to the acid-binding agent is 1:0.1∼0.5;
(3) post-processing: cooling the aforementioned reaction solution to room temperature, adding water to dissolve the hydrochloride of acid-binding agent, filtering, and layering the filtrate, separating the water layer, extracting the organic layer with aqueous acid at least three times, then combining the aqueous acid layer after the extraction and adding water to dilute, crystallizing, filtering to give wet 2,3-dichloropyridine, drying the wet product to give the product of 2,3-dichloropyridine.

2. The method as defined in claim 1,
wherein the catalyst is selected from sulfur trioxide, boron trifluoride, ferric chloride or aluminum chloride, the amount of the catalyst is 0.01 to 1.00 times the molar amount of 2,6-dichloropyridine in the chlorination;
wherein the temperature is 100∼200°C in the chlorination;
wherein the distillate with low concentration gained during the process of fractionating of said chlorination can be recycled for the next batch of reaction and rectification in the chlorination.

3. The method as defined in claim 2,
wherein the amount of the catalyst is 0.01 to 0.50 times the molar amount of 2,6-dichloropyridine in the chlorination.

4. The method as defined in claim 1,
wherein the acid-binding agent of the hydrogenation is selected from organic base or inorganic base compound; the organic base compound is selected from pyridine, triethylamine, alkaline metal salts of alcohols, alkyl lithium compound or N-lithium compound; the inorganic base compound is selected from the group consisting of alkaline metal hydroxides, alkaline metal carbonates and bicarbonates, acetates;
wherein the metal catalyst of the hydrogenation is selected from mono-metallic catalyst, multi-metal catalyst, supported metal catalyst or non-supported metal catalyst;
wherein the organic solvent of the hydrogenation is selected from esters, ethers, aromatic hydrocarbons, nitriles, or any mixture of them.

5. The method as defined in claim 4,
wherein the alkaline metal salts of alcohols is selected from sodium methylate, potassium ethylate, or potassium tert-butoxide; the alkyl lithium compound is selected from butyl lithium or phenyl lithium; the N-lithium compound is selected from lithium diisopropyl amide or lithium hexamethyldisilazide; the alkaline metal hydroxides is selected from sodium hydroxide or potassium hydroxide; the alkaline metal carbonates and bicarbonates is selected from sodium carbonate, potassium carbonate, sodium bicarbonate or potassium bicarbonate; the acetates is selected from sodium acetate or potassium acetate;
the mono-metallic catalyst is selected from platinum, palladium, cobalt or nickel; the multi-metal catalyst is selected from one of the binary alloy catalysts which is copper-nickel, copper-palladium, palladium-silver, platinum-gold or platinum-copper; the supported metal catalyst is selected from platinum carbon or palladium-carbon; the non-supported metal catalyst is selected from Raney nickel, Raney copper or Raney cobalt;
the esters is selected from methyl acetate, ethyl acetate or butyl acetate; the ethers is selected from diethyl ether, methyl tert-butyl ether, tetrahydrofuran or dioxane, the aromatic hydrocarbons is selected from benzene, toluene or chlorobenzene, the nitriles is selected from acetonitrile or propionitrile.

6. The method as defined in claim 4 or claim 5,
wherein the acid-binding agent is selected from pyridine or triethylamine;
wherein the metal catalyst is selected from platinum carbon, palladium-carbon, Raney nickel or Raney copper;
wherein the organic solvent is selected from toluene or ethyl acetate.

7. The method as defined in claim 1,
wherein the pressure range in the hydrogenation is 0-5Mpa;
wherein the temperature of the hydrogenation is 20-150°C;
wherein the molar ratio of the 2,3,6-trichloropyridine to acid-binding agent in the hydrogenation is 1:0.3∼0.5.

8. The method as defined in claim 1,
wherein the catalyst gained by filtration after water is added to dissolve the hydrochloride of acid-binding agent can be recycled in the post-processing;
wherein the organic solvent layer can be recycled as material for the next batch in the described post-processing when replenishing 2,3,6-trichloropyridine and organic solvent;
wherein the aqueous acid is selected from water-soluble inorganic acid or organic acid; the inorganic acid is selected from hydrochloric acid, sulfuric acid, carbonic acid, phosphoric acid or nitric acid; the organic acid is selected from low-level carboxylic acid: formic acid, acetic acid, propionic acid, n-butyric acid or n-pentanoic acid in the post-processing;
wherein the molar ratio of the aqueous acid to 2,3,6-trichloropyridine is 1∼5:1.

9. The method as defined in claim 8, wherein the organic acid can also be selected from salicylic acid or benzenesulfonic acid.

## Patentansprüche

1. Verfahren zur Herstellung von 2,3-Dichlorpyridin, welches die folgenden Schritte umfasst:
(1) Chlorierung: Vermischen des Ausgangsmaterials 2,6-Dichlorpyridin mit einem Katalysator, dann Erwärmen und Einleiten von Chlorgas zum Chlorieren, wenn die Reaktion beendet ist, Abkühlen und Fraktionieren unter verringertem Druck, um 2,3,6-Trichlorpyridin zu erhalten;
(2) Hydrierung: Hinzufügen von 2,3,6-Trichlorpyridin, welches durch die Chlorierung erhalten wurde, eines Säurebindemittels, eines Metallkatalysators und eines organischen Lösungsmittels in einen Reaktor unter dem Druckbereich von 0 - 10 MPa, Einleiten von Wasserstoffgas zur Hydrierung nach dem Erwärmen, wenn der pH-Wert der Reaktionslösung 4∼8 erreicht hat, Stoppen der Einleitung des Wasserstoffgases; das Molverhältnis des 2,3,6-Trichlorpyridin zu dem Säurebindemittel ist 1:0,1∼0,5;
(3) Nachbearbeitung: Abkühlen der zuvor genannten Reaktionslösung auf Raumtemperatur, Hinzufügen von Wasser, um das Hydrochlorid von dem Säurebindemittel zu trennen, Filtern und Schichten des Filtrats, Trennen der Wasserschicht, Extrahieren der organischen Schicht mit wässriger Säure zumindest drei Mal, dann Binden der wässrigen Säureschicht nach der Extraktion und Hinzufügen von Wasser zum Verdünnen, Kristallisieren, Filtern, um nasses 2,3-Dichlorpyridin zu erhalten, Trocknen des nassen Produkts, um das Produkt aus 2,3-Dichlorpyridin zu erhalten.

2. Verfahren nach Anspruch 1,
wobei der Katalysator aus Schwefeltrioxid, Bortrifluorid, Eisenchlorid oder Aluminiumchlorid ausgewählt wird, wobei die Menge des Katalysators 0,01 bis 1,00 Mal die molare Menge von 2,6-Dichlorpyridin in der Chlorierung ist;
wobei die Temperatur 100∼200°C in der Chlorierung ist;
wobei das Destillat mit niedriger Konzentration, welches während des Fraktionierungsprozesses der Chlorierung erhalten wurde, für den nächsten Reaktions- und Rektifikationsansatz bei der Chlorierung wiederverwendet werden kann.

3. Verfahren nach Anspruch 2,
wobei die Menge des Katalysators 0,01 bis 0,50 Mal die molare Menge von 2,6-Dichlorpyridin bei der Chlorierung ist.

4. Verfahren nach Anspruch 1,
wobei das Säurebindemittel der Hydrierung aus einer organischen Basenverbindung oder einer anorganischen Basenverbindung ausgewählt wird; die organische Basenverbindung wird aus Pyridin, Triethylamin, alkalischen Metallsalzen von Alkoholen, Alkyllithiumverbindung oder N-Lithiumverbindung ausgewählt; die anorganische Basenverbindung wird von der Gruppe bestehend aus alkalischen Metallhydroxiden, alkalischen Metallcarbonaten und -bicarbonaten, Acetaten ausgewählt;
wobei der Metallkatalysator der Hydrierung aus einem mono-metallischem Katalysator, multi-Metallkatalysator, getragenen Metallkatalysator oder einem nichtgetragenen Metallkatalysator ausgewählt wird;
wobei die organische Lösung der Hydrierung aus Ester, Ether, aromatischen Kohlenwasserstoffen, Nitril oder einem Gemisch von diesen ausgewählt wird.

5. Verfahren nach Anspruch 4,
wobei die alkalischen Metallsalze von Alkoholen aus Natriummethylat, Kaliumethylat oder Kalium-*tert*-Butoxid ausgewählt wird; die Alkyllithiumverbindung wird aus Butyllithium oder Phenyllithium ausgewählt; die N-Lithiumverbindung wird aus Lithiumdiisopropylamid oder Lithiumhexamethyldisilazid ausgewählt; die alkalischen Metallhydroxide werden aus Natriumhydroxid oder Kaliumhydroxid ausgewählt; die alkalischen Metallcarbonate und -bicarbonate werden aus Natriumcarbonat, Kaliumcarbonat, Natriumbicarbonat oder Kaliumbicarbonat ausgewählt; die Acetate werden aus Natriumacetat oder Kaliumacetat ausgewählt;
der mono-metallische Katalysator wird aus Platin, Palladium, Kobalt oder Nickel ausgewählt; der multi-Metallkatalysator wird aus einem der binären Legierungskatalysatoren, welche Kupfer-Nickel, Kupfer-Palladium, Palladium-Silber, Platin-Gold oder Platin-Kupfer sind, ausgewählt; der getragene Metallkatalysator wird aus Platincarbon oder Palladiumcarbon ausgewählt; der nichtgetragene Metallkatalysator wird aus Raneynickel, Raneykupfer oder Raneykobalt ausgewählt;
die Ester werden aus Methylacetat, Ethylacetat oder Butylacetat ausgewählt; die Ether werden aus Diethylether, Methyl-*tert*-Butylether, Tetrahydrofuran oder Dioxan ausgewählt, die aromatischen Kohlenwasserstoffe werden aus Benzen, Toluen oder Chlorbenzen ausgewählt, die Nitrile werden aus Acetonitril oder Propionitril ausgewählt.

6. Verfahren nach Anspruch 4 oder 5,
wobei das Säurebindemittel aus Pyridin oder Triethylamin ausgewählt wird,
wobei der Metallkatalysator aus Platincarbon, Palladium-Carbon, Raneynickel oder Raneykupfer ausgewält wird,
wobei die organische Lösung aus Toluen oder Ethylacetat ausgewählt wird.

7. Verfahren nach Anspruch 1,
wobei der Druckbereich bei der Hydrierung 0-5 MPa ist;
wobei die Temperatur der Hydrierung 20-150°C ist;
wobei das Molverhältnis des 2,3,6-Trichlorpyridin zu dem Säurebindemittel bei der Hydrierung 1:0,3∼0,5 ist.

8. Verfahren nach Anspruch 1,
wobei der Katalysator, welcher durch Filtration erhalten wurde, nachdem Wasser hinzugefügt wurde, um das Hydrochlorid des Säurebindemittels zu lösen, in der Nachbehandlung wiederaufbereitet werden kann;
wobei die organische Lösungsmittelschicht als Material für den nächsten Ansatz in der beschriebenen Nachbehandlung wiederverwendet werden kann, wenn das 2,3,6-Trichlorpyridin und das organische Lösemittel wieder aufgefüllt werden;
wobei die wässrige Säure aus wasserlöslicher anorganischer Säure oder organischer Säure ausgewählt wird; die anorganische Säure wird aus Chlorwasserstoffsäure, Schwefelsäure, Kohlensäure, Phosphorsäure oder Salpetersäure ausgewählt; die organische Säure wird aus niedriger Carbonsäure: Methansäure, Ethansäure, Propansäure, n-Butansäure oder n-Pentansäure in der Nachbehandlung ausgewählt; wobei das Molverhältnis der wässrigen Säure bei 2,3,6-Trichlorpyridin 1∼5:1 ist.

9. Verfahren nach Anspruch 8, wobei die organische Säure auch aus Salizylsäure oder Benzolsulfonsäure ausgewählt werden kann.

## Revendications

1. Procédé de préparation de 2,3-dichloropyridine, qui comprend les étapes suivantes :
(1) chloration : mélange de la matière première de départ 2,6-dichloropyridine avec un catalyseur, puis chauffage et introduction de chlore gazeux en vue de la chloration, lorsque la réaction est terminée, refroidissement et fractionnement sous pression réduite pour donner de la 2,3,6-trichloropyridine ;
(2) hydrogénation : ajout de la 2,3,6-trichloropyridine obtenue par la chloration, d'un agent fixateur d'acide, d'un catalyseur métallique et d'un solvant organique dans un réacteur, sous une pression dans la plage de 0 à 10 MPa, introduction d'hydrogène gazeux en vue de l'hydrogénation après chauffage, lorsque la valeur de pH de la solution de réaction a atteint 4∼8, arrêt de l'introduction de l'hydrogène gazeux ; le rapport molaire de ladite 2,3,6-trichloropyridine à l'agent fixateur d'acide est de 1/0,1∼0,5 ;
(3) post-traitement : refroidissement de la solution de réaction mentionnée ci-dessus à température ambiante, ajout d'eau pour dissoudre le chlorhydrate de l'agent fixateur d'acide, filtration, et dépôt du filtrat, séparation de la phase aqueuse, extraction de la phase organique avec de l'acide aqueux au moins trois fois, puis combinaison de la phase d'acide aqueux après l'extraction et ajout d'eau en vue de la dilution, cristallisation, filtration pour donner de la 2,3-dichloropyridine humide, séchage du produit humide pour donner le produit de 2,3-dichloropyridine.

2. Procédé tel que défini dans la revendication 1,
dans lequel le catalyseur est choisi parmi le trioxyde de soufre, le trifluorure de bore, le chlorure ferrique ou le chlorure d'aluminium, la quantité du catalyseur est de 0,01 à 1,00 fois la quantité molaire de 2,6-dichloropyridine dans la chloration ;
dans lequel la température est de 100 à 200°C dans la chloration ;
dans lequel le distillat à faible concentration obtenu au cours du procédé de fractionnement de ladite chloration peut être recyclé pour la série suivante de réaction et de rectification dans la chloration.

3. Procédé tel que défini dans la revendication 2,
dans lequel la quantité du catalyseur est de 0,01 à 0,50 fois la quantité molaire de 2,6-dichloropyridine dans la chloration.

4. Procédé tel que défini dans la revendication 1,
dans lequel l'agent fixateur d'acide de l'hydrogénation est choisi parmi un composé de type base organique ou de type base inorganique ; le composé de type base organique est choisi parmi la pyridine, la triéthylamine, les sels de métaux alcalins d'alcools, un composé alkyl-lithium ou un composé N-lithium ; le composé de type base inorganique est choisi dans le groupe constitué des hydroxydes de métaux alcalins, des carbonates et bicarbonates de métaux alcalins, des acétates ;
dans lequel le catalyseur métallique de l'hydrogénation est choisi parmi un catalyseur mono-métallique, un catalyseur multi-métallique, un catalyseur métallique supporté ou un catalyseur métallique non supporté ;
dans lequel le solvant organique de l'hydrogénation est choisi parmi les esters, les éthers, les hydrocarbures aromatiques, les nitriles, ou tout mélange de ceux-ci.

5. Procédé tel que défini dans la revendication 4,
dans lequel les sels de métaux alcalins d'alcools sont choisis parmi le méthylate de sodium, l'éthylate de potassium ou le tert-butoxyde de potassium ; le composé alkyl-lithium est choisi parmi le butyl-lithium ou le phényl-lithium ; le composé N-lithium est choisi parmi le diisopropylamide de lithium ou l'hexaméthyldisilazide de lithium ; les hydroxydes de métaux alcalins sont choisis parmi l'hydroxyde de sodium ou l'hydroxyde de potassium ; les carbonates et bicarbonates de métaux alcalins sont choisis parmi le carbonate de sodium, le carbonate de potassium, le bicarbonate de sodium ou le bicarbonate de potassium ; les acétates sont choisis parmi l'acétate de sodium ou l'acétate de potassium ;
le catalyseur mono-métallique est choisi parmi le platine, le palladium, le cobalt ou le nickel ; le catalyseur multi-métallique est choisi parmi l'un des catalyseurs de type alliage binaire constitués du cuivre-nickel, du cuivre-palladium, du palladium-argent, du platine-or ou du platine-cuivre ; le catalyseur métallique supporté est choisi parmi le platine-carbone ou le palladium-carbone ; le catalyseur métallique non supporté est choisi parmi le nickel de Raney, le cuivre de Raney ou le cobalt de Raney ;
les esters sont choisis parmi l'acétate de méthyle, l'acétate d'éthyle ou l'acétate de butyle ; les éthers sont choisis parmi l'éther diéthylique, l'éther méthyl tert-butylique, le tétrahydrofuranne ou le dioxane, les hydrocarbures aromatiques sont choisis parmi le benzène, le toluène ou le chlorobenzène, les nitriles sont choisis parmi l'acétonitrile ou le propionitrile.

6. Procédé tel que défini dans la revendication 4 ou la revendication 5,
dans lequel l'agent fixateur d'acide est choisi parmi la pyridine ou la triéthylamine ;
dans lequel le catalyseur métallique est choisi parmi le platine-carbone, le palladium-carbone, le nickel de Raney ou le cuivre de Raney ;
dans lequel le solvant organique est choisi parmi le toluène ou l'acétate d'éthyle.

7. Procédé tel que défini dans la revendication 1,
dans lequel la plage de pression dans l'hydrogénation est de 0 à 5 MPa ;
dans lequel la température de l'hydrogénation est de 20 à 150°C ;
dans lequel le rapport molaire de la 2,3,6-trichloropyridine à l'agent fixateur d'acide dans l'hydrogénation est de 1/0,3∼0,5.

8. Procédé tel que défini dans la revendication 1,
dans lequel le catalyseur obtenu par filtration après que l'eau est ajoutée pour dissoudre le chlorhydrate de l'agent fixateur d'acide peut être recyclé dans le post-traitement ;
dans lequel la phase de solvant organique peut être recyclée en tant que produit pour la série suivante dans le post-traitement décrit au cours de la recharge en 2,3,6-trichloropyridine et en solvant organique ;
dans lequel l'acide aqueux est choisi parmi un acide inorganique ou un acide organique soluble dans l'eau ; l'acide inorganique est choisi parmi l'acide chlorhydrique, l'acide sulfurique, l'acide carbonique, l'acide phosphorique ou l'acide nitrique ; l'acide organique est choisi parmi l'acide carboxylique à faible teneur : l'acide formique, l'acide acétique, l'acide propionique, l'acide n-butyrique ou l'acide n-pentano'ique dans le post-traitement ; dans lequel le rapport molaire de l'acide aqueux à la 2,3,6-trichloropyridine est de 1∼5/1.

9. Procédé tel que défini dans la revendication 8, dans lequel l'acide organique peut également être choisi parmi l'acide salicylique ou l'acide benzènesulfonique.
